# EUROPEAN PATENT APPLICATION

(11) **EP 2 016 938 A1**
(43) Date of publication of application: **21.01.2009**
(21) Application number: 08075760.2
(22) Date of filing: 25.09.2002
(51) Int. Cl.: A61K 9/51

(54) **Preparation of submicron sized particles via dispersion and solevnt or liquid phase removal**

(30) Priority: 26.09.2001 US 964273; 26.06.2002 US 183035
(62) Divisional of application: 02773579.4
(71) Applicant: Baxter International Inc., Deerfield, IL 60015 (US)
(72) Inventor: Brynjelsen, Sean, Lake-In-The-Hills, Illinois 60156 (US); Sternberg, Shmuel, Palatine, Illinois 60067 (US); Dunham, Andrew J., Round Lake, Illinois 60073 (US); Narayanan, Krishnaswamy, Waukesha, Wisconsin 53188 (US); Doty, Mark J., Grayslake, Illinois 60030 (US); Kipp, James E, Wauconda, Illinois 60084 (US); Jayswal, Nailesh, Bensenville, Illinois 60106 (US)
(74) Representative: Dee, Ian Mark

(57) **Abstract**

The present invention relates to a process for preparing submicron sized particles comprising the steps of: providing a crude dispersion of a multiphase system having an organic phase and an aqueous phase, the organic phase having a pharmaceutically active compound therein; providing energy to the crude dispersion to form a fine dispersion; freezing the fine dispersion; and lyophilizing the frozen dispersion to obtain particles having a mean particle size of less than 500nm.

## Description

### CROSS REFERENCE TO RELATED APPLICATIONS:

This application claims the benefit of co-pending U.S. Patent Application Serial No. 09/964,273, filed September 26, 2001, and U.S. Patent Application Serial No. 10/183,035, filed June 26, 2002.
FEDERALLY SPONSORED RESEARCH OR DEVELOPMENT:
   Not Applicable.

### BACKGROUND OF THE INVENTION:

### Technical Field

The present invention relates to a process for preparing submicron sized nanoparticles of a poorly water soluble compound by lyophilizing a dispersion of a multiphase system having an organic phase and an aqueous phase, the organic phase having the poorly water soluble compound therein. The method is preferably used to prepare nanoparticles of a poorly water soluble, pharmaceutically active compound suitable for *in vivo* delivery, particularly by parenteral routes.

### Background of the Invention

There are an ever increasing number of pharmaceutical drugs being formulated that are poorly soluble or insoluble in aqueous solutions. Such drugs provide challenges to delivering them in an injectable form such as through parenteral administration. Drugs that are insoluble in water can have significant benefits when formulated as a stable suspension of submicron sized particles. Accurate control of particle size is essential for safe and efficacious use of these formulations.

Particles must be less than seven microns in diameter to safely pass through capillaries without causing emboli (Allen et al., 1987; Davis and Taube, 1978; Schroeder et al., 1978; Yokel et al., 1981). One solution to this problem is the production of extremely small particles of the insoluble drug candidate and the creation of a microparticulate or nanoparticulate suspension. In this way, drugs that were previously unable to be formulated in an aqueous based system can be made suitable for parenteral administration. Suitability for parenteral administration includes small particle size (<7 µm), low toxicity (as from toxic formulation components or residual solvents), and bioavailability of the drug particles after administration.

The parenteral administration of such poorly water soluble pharmaceutical agents has been achieved in the past using emulsions composed of a hydrophobic solvent (e.g., oil) and a stabilized drug dispersed within an aqueous medium, such as a buffer solution or normal saline solution. These liquid/liquid emulsions may be injected intravenously.

One example of this approach utilized the anesthetic, propofol (2,6 diisopropylphenol), in which the pharmacological agent was dissolved within a vegetable oil emulsion to enable intravenous administration. See, e.g., U.S. Pat. Nos. 4,056,635; 4,452,817 and 4,798,846, all to Glen et al. Such emulsions, however, tend to be unstable given the predominance of the oil phase and the absence of antimicrobial agents. In other instances, even where the pharmacological agent is successfully incorporated into an oil-free formulation, particles containing the pharmacological agent may cause irritation at the site of delivery because of their size or form. Furthermore, many insoluble drugs of interest do not show appreciable solubility within traditional oil emulsion systems. One reason for this is that solubility is not strictly defined by polarity, but also includes hydrogen bonding, dipole-dipole interactions, ionic stabilization and atom to atom interactions.

U.S. Pat. No. 4,073,943, issued to Wretlind et al., discloses a method of administering a water-insoluble pharmaceutically active agent by dissolving the agent in oil and emulsifying the solution with water in the presence of surfactants (egg phosphatides, pluronics, polyglycerol oleate, etc.) to form stable lipoid particles of the agent dispersed in the aqueous phase.

U.S. Pat. No. 4,540,602, issued to Motoyama et al., discloses a process for the preparation of an activated pharmaceutical composition containing a water insoluble drug for oral administration. In one procedure of the invention (see Examples 4 to 10), the process is carried out by dissolving the drug in hydrophobic organic solvents, and the resulting solution is emulsified in water. The dispersing medium is then removed rapidly by spray drying, resulting in particles ranging in particle size of from about 0.1 to about 3.0 µm.

A variety of approaches have been explored for developing stable formulations of a substantially water-insoluble pharmacologically active agent for *in vivo* delivery. One approach is directed to the production of suspended particles coated with protein. U.S. Pat. 5,916,596, issued to Desai et al., discloses the application of high shear to a mixture of an organic phase having a pharmacologically active agent dispersed therein and an aqueous medium containing a biocompatible polymer. The mixture is sheared in a high pressure homogenizer at a pressure in the range of from about 3,000 to 30,000 psi. The '596 patent provides that the mixture must contain substantially no surfactants because the combined use of a surfactant with a protein results in the formation of large, needle-like crystalline particles that increase in size during storage. See columns 17-18, example 4. The biocompatible polymer may be crosslinked as the result of exposure to the high shear conditions in a high pressure homogenizer. In the embodiment in which protein containing sulfhydryl or disulfide groups is used (e.g. albumin), the protein forms a crosslinked shell around droplets of non-aqueous medium. See Column 8, lines 35-48. In Examples 1, 2, 5, 6, 9, 10, 11, and 12, the organic phase is removed rapidly by rotary evaporation at 40°C and at a reduced pressure of 30 mm Hg, resulting in an aqueous dispersion of particles coated with crosslinked protein. The aqueous dispersion may further be lyophilized to remove the aqueous phase. The '596 patent discloses other alternative methods of removing the solvent, including falling film evaporation, spray drying, and freeze-drying. Example 2 discloses that the crude emulsion may be sonicated to produce nanoparticles ranging from 350-420 nanometers. Example 5 discloses a method to prepare sterile-filterable nanoparticles of less than 200 nm. This method requires that the pharmaceutical agent is initially dissolved in a mixture of substantially water immiscible organic solvent (e.g., chloroform) and a water miscible organic solvent (e.g. ethanol).

U.S. Pat. No. 5,560,933, issued to Soon-Shiong et al., discloses the formation of a polymeric shell around the water-insoluble oil (containing the drug) for *in vivo* delivery. The method discloses the application of sonication to a mixture comprising a polymer-containing aqueous medium and a dispersing agent (oil) having a substantially water-insoluble drug dispersed therein. In this reference, sonication is used to drive the formation of disulfide bonds in the polymer, causing it to crosslink so as to produce a polymeric shell around the drug. Sonication is conducted for a time sufficient for the disulfide bonds to form.

In U.S. Pat. No. 5,665,383, Grinstaff et al. discloses the application of ultrasound to a single-phase, i.e., an aqueous medium, to encapsulate an immunostimulating agent within a polymeric shell for *in vivo* delivery. The ultrasound promotes crosslinking of the encapsulating agent by disulfide bonds to form the shell.

Another approach to preparing a water-insoluble drug for *in vivo* delivery centers on reducing the size of the particles that deliver the drug. In one such series of patents, which include U.S. Pat. Nos. 6,228,399; 6,086,376; 5,922,355; and 5,660,858, Parikh et al*.* discloses that sonication may be used to prepare microparticles of the water-insoluble compound. Of these patents, U.S. Pat. No. 5,922,355 discloses an improvement to a method that uses sonication for making the smaller particles. The improvement comprises mixing an active pharmacological agent with a phospholipid and surfactants in a single-phase aqueous system and applying energy to the system to produce the smaller particles.

U.S. Pat. No. 5,091,188, issued to Haynes, also discloses reducing the size of particles of a pharmacologically active water-insoluble drug and employing a lipid coating on the particles to confer a solid form. The patent is directed to a pharmaceutical composition of an aqueous suspension of solid particles of the drug having a diameter of about 0.05 to about 10 microns. The lipid coating affixed to the surface of the particles contributes to their solid form. The composition is produced by adding the drug to water and then reducing the particle size within the aqueous suspension. Example 6 of this reference discloses the use of a pharmacologically acceptable oil, which is selected for its inability to dissolve the crystalline drug. See column 16, lines 8-12.

Still another approach for preparing microparticles of a pharmacological agent focuses on the use of phase inversion principles. U.S. Pat. Nos. 6,235,224 B1 and 6,143,211, both issued to Mathiowitz et al., disclose the use of phase inversion phenomena to precipitate microencapsulated microparticles. The method includes mixing a polymer and a drug with a solvent. This mixture is introduced into an effective amount of a miscible nonsolvent, thereby causing spontaneous formation of the microencapsulated product.

Microprecipitation by pH shifting is another technology used to prepare dispersions of a nanoparticulate pharmaceutical agent. See, e.g., U.S. Pat. Nos. 5,665,331; and 5,662,883. This technology involves dissolving a pharmaceutical in an aqueous base that is then neutralized to form a dispersion.

In yet another approach, such as that disclosed in U.S. Pat. No. 5,766,635, issued to Spenlenhauer et al., nanoparticles have been prepared by dissolving a poly(ethylene) oxide and/or poly(propylene) oxide in an organic solvent, mixing the organic solution so formed with an aqueous solution to cause nanoparticles to precipitate out of solution, and microfluidizing the precipitated solution without the use of surfactants.

The commonly assigned and co-pending U.S. Application Serial Nos. 09/874,499; 09/874,799; 09/874,637; 09/953,979; and 10/021,692, which are incorporated herein by reference and made a part hereof, disclose a process for preparing submicron particles by microprecipitation. The process disclosed includes the steps of: (1) dissolving an organic compound in a water miscible first organic solvent to create a first solution; (2) mixing the first solution with a second solvent of water to precipitate the organic compound to create a presuspension; and (3) adding energy to the presuspension in the form of high-shear mixing or heat to provide a stable form of the organic compound having the desired size ranges. One or more optional surface modifiers can be added to the first organic solvent or the second aqueous solution.

The commonly assigned and co-pending U.S. Application Serial No. 09/964,273, which is incorporated herein by reference and made a part hereof, discloses a process for preparing submicron particles by sonicating a crude emulsion of a multiphase phase system having an organic phase and an aqueous phase, the organic phase having a pharmaceutically active compound therein. The organic phase is evaporated from the emulsion under specific sonication conditions in order to generate the drug particles. The particle formed by this process typically has an average effective particle size of less than 2 µm.

Because of the difficulties posed by poorly soluble drugs in drug therapy, the need for new technologies continues to expand for addressing these problems.

### SUMMARY OF THE INVENTION:

The present invention provides a method for preparing submicron sized particles. The method includes the steps of: (1) providing a multiphase system having a liquid phase comprising an organic phase and an aqueous phase, the organic phase having a pharmaceutically active compound therein; and (2) removing a portion of the organic phase or the liquid phase of the multiphase system to obtain submicron sized particles of the conpound.

In one aspect, the present invention provides a method for preparing submicron sized particles which includes the steps of: (1) providing a multiphase system having an organic phase and an aqueous phase, the organic phase having a pharmaceutically active compound therein; and (2) sonicating the system to evaporate a portion of the organic phase to cause precipitation of the compound in the aqueous phase and having an average effective particle size of less than about 2 µm.

In another aspect, the present invention provides a process for preparing an aqueous suspension of submicron sized particles which includes the steps of: (1) providing an organic phase of a pharmaceutically active compound dissolved in a water immiscible solvent; (2) providing an aqueous phase; (3) combining the organic phase with the aqueous phase to form a crude dispersion; and (4) sonicating the crude dispersion to cause precipitation of the compound as a fine dispersion of particles in the aqueous phase wherein the aqueous phase is essentially free of the water immiscible solvent.

In yet another aspect, the present invention provides a process for preparing submicron sized particles of a pharmaceutically active compound which includes the steps of: (1) providing a crude dispersion of a multiphase system having an organic phase and an aqueous phase, the organic phase having a pharmaceutically active compound therein; (2) providing energy to the crude dispersion to form a microdroplet or fine dispersion; (3) freezing the fine dispersion; and (4) lyophilizing the frozen dispersion to obtain particles having a mean particle size of less than 500 nm. In a preferred embodiment, the particles have a mean particle size of less than 200 nm.

In still yet another aspect, the present invention provides a process for preparing submicron sized particles of a pharmaceutically active compound which includes the steps of: (1) providing an organic phase of a pharmaceutically active compound dissolved in a water immiscible solvent; (2) providing an aqueous phase; (3) combining the organic phase with the aqueous phase to form a crude dispersion; (4) providing energy to the crude dispersion to form a microdroplet or fine dispersion; (5) freezing the fine dispersion; and (6) lyophilizing the frozen dispersion to obtain particles having a mean particle size of less than 500 nm. In a preferred embodiment, the particles have a mean particle size of less than 200 nm.

These and other aspects and attributes of the present invention will be discussed with reference to the following drawings and accompanying specification.

### BRIEF DESCRIPTION OF THE DRAWINGS:

FIG. 1 is a schematic representation of a process of preparing submicron sized particles in a multiphase system by sonicating the system to evaporate a portion of the organic phase;
FIG. 2A is a schematic representation of a process of of preparing submicron sized particles in a multiphase system by freezing and lyophilizing a dispersion; FIG. 2B is a schematic diagram of the steps of freezing and lyophilizing the dispersion to obtain submicron sized particles of the present invention.
FIG. 3 is a high magnification SEM photograph of itraconazole particles;
FIG. 4 is a low magnification SEM photograph of itraconazole particles; and
FIG. 5 is an x-ray powder diffraction spectrum of itraconazole nanoparticles and raw material.

### DETAILED DESCRIPTION OF THE PREFERRED EMBODIMENT

While this invention is susceptible of embodiment in many different forms, there is shown in the drawing, and will be described herein in detail, specific embodiments thereof with the understanding that the present disclosure is to be considered as an exemplification of the principles of the invention and is not intended to limit the invention to the specific embodiments illustrated.

The present invention relates to a process for preparing submicron sized particle dispersions which includes the steps of: (1) providing a multiphase system having a liquid phase comprising an organic phase and an aqueous phase, the organic phase having a pharmaceutically active compound herein, and (2) removing a portion of the organic phase or the liquid phase of the multiphase system to form submicron sized particles of the compound.

In one aspect, the present invention provides a process for preparing submicron-sized particle dispersions. The process comprises the steps of: (1) providing a multiphase system having an organic phase and an aqueous phase, the organic phase having a pharmaceutically active compound therein; and (2) sonicating the system to evaporate a portion of the organic phase to cause precipitation of the compound in the aqueous phase and having an average effective particle size of less than about 2 µm.

In another aspect, the present invention provides a process for preparing submicron-sized nanoparticles. The process includes the steps of: (1) providing a crude dispersion of a multiphase system having an organic phase and an aqueous phase, the organic phase having a pharmaceutically active compound with or without surface active compounds dissolved therein; (2) providing energy conditions to the crude dispersion to form a fine dispersion having an average effective oil droplet size of less than about 2 µm; (3) freezing the fine dispersion so that the bulk phase is minimally in a frozen state; and (4) lyophilizing the dispersion to obtain nanoparticles having a mean particle size of less than 500 nm, and preferably less than 200 nm.

The preferred step of providing the multiphase system includes the steps of: (1) mixing a water immiscible solvent with the pharmaceutically active compound to define an organic solution, (2) preparing an aqueous based solution, and (3) mixing the organic solution with the aqueous solution to form the multiphase dispersion system. The water immiscible solvent used may be partially water miscible and the organic solution in the system may further include a co-solvent. The multiphase system may also include one or more surface active compounds which can be added to the aqueous based solution, or to the organic solution, or to the both the aqueous solution and the organic solution, or to the mixture of the aqueous solution and the organic solution. The multiphase system can be agitated or mixed to form a crude dispersion. The crude dispersion can also be formed by any other low-energy input processes known in the art, such as shaking, vortexing, agitating, mixing (e.g. Ultraturrax) or stirring. In a preferred embodiment of the invention, the crude dispersion is formed by mixing the multiphase system with an Ultraturrax-T25 mixer for 10 seconds. The crude dispersion will have oil droplets in the water of a size of approximately less than 2 µm in diameter. The crude dispersion is subjected to addition of energy to define a microdispersion or submicron oil in water suspension. Examples of methods for providing energy to the crude dispersion include sonication, homogenization, microfluidization or other appropriate high shear techniques.

What is meant by the term "multiphase system" is a dispersion having at least one organic phase and at least one aqueous phase and in a preferred form of the invention is an oil in water (O/W) emulsion where the water phase forms the continuous phase and the oil phase forms the dispersed phase. The organic phase is preferably a water immiscible or a partially water miscible organic solvent. The organic phase may also include a co-solvent for the pharmaceutically active compound. A preferred co-solvent is a water miscible organic solvent, such as ethanol, methanol, and acetone. The ratio by weights of the organic phase to the aqueous phase is from about 1:99 to about 99:1, more preferably from 1:99 to about 40:60, and most preferably from about 2:98 to about 30:70, or any range or combination of ranges therein. The present invention further contemplates utilizing reverse emulsions or water in oil emulsion (W/O) where the oil phase forms the continuous phase and water the dispersed phase. The present invention further contemplates utilizing emulsions having more than two phases such as an oil in water in oil emulsion (O/W/O) or a water in oil in water emulsion (W/O/W). The present invention is intended in forming a liquid in liquid dispersion multiphase system. Submicron sized particles are formed when the liquid phases of the multiphase system are removed by, for example, lyophilization. Furthermore, such a dispersion system can be sterile filtered. However, solid particles may be formed during the process of forming the crude dispersion or the fine dispersion. These solid particles may be dispersed in the organic phase and/or the aqueous phase of the multiphase system.

What is meant by the term "pharmaceutically active compound" is any compound that has therapeutic effect and more particularly to such compounds that are insoluble or slightly soluble in water with a solubility of preferably less than 10 mg/ml, and more preferably less than 8 mg/ml. Such compounds can be found in the Physicians' Desk Reference. Particularly suitable pharmaceutically active compounds include, but are not limited to, antihyperlipidemics; antimicrobials, e.g., antibacterials such as sulfadiazine, antifungals such as itraconazole; non-steroidal anti-inflammatory drugs, e.g., indomethacin; antihypercholesteremic agents, e.g., probucol; and steroidal compounds, e.g., dexamethasone; immunosuppresants, e.g., cyclosporin A, tacrolimus, and mycophenolate mofetil. Or the organic compound might be from the group used as adjuvants or excipients in pharmaceutical preparations and cosmetics, such as, but not limited to, preservatives, e.g., propylparaben.

The pharmaceutically active compound can be present in a concentration to the extent it is soluble in the organic phase. In a preferred form of the invention the pharmaceutically active compound can be present in an amount from less than 1 % to about 40%, more preferably from about 1% to about 25%, and most preferably from about 1% to about 10% by weight of the organic phase, or any range or combination of ranges therein.

What is meant by the term "water immiscible solvent" are those solvents which form an interfacial meniscus when combined with an aqueous solution in a 1:1 ratio (o/w). In a preferred form of the invention the water immiscible solvent will have a vapor pressure higher than that of water when both the solvent and water are measured at room temperature. Suitable water immiscible solvents include, but are not limited to, substituted or unsubstituted, linear, branched or cyclic alkanes with a carbon number of 5 or higher, substituted or unsubstituted, linear, branched or cyclic alkenes with a carbon number of 5 or higher, substituted or unsubstituted, linear, branched or cyclic alkynes with a carbon number of 5 or higher; aromatic hydrocarbons completely or partially halogenated hydrocarbons, ethers, esters, ketones, mono-, di- or tri-glycerides, native oils, alcohols, aldehydes, acids, amines, linear or cyclic silicones, hexamethyldisiloxane, or any combination of these solvents. Halogenated solvents include, but are not limited to carbon tetrachloride, methylene chloride, chloroform, tetrachloroethylene, trichloroethylene, trichloroethane, hydrofluorocarbons, chlorinated benzene (mono, di, tri), trichlorofluoromethane. Particularly suitable solvents are methylene chloride, chloroform, diethyl ether, toluene, xylene and ethyl acetate. What is meant by "partially water miscible solvents" are those solvents which are water immiscible at one concentration, and water miscible at another lower concentration. These solvents are of limited water miscibility and capable of spontaneous emulsion formation. Examples of partially water miscible solvents are tetrahydrofuran (THF), propylene carbonate, benzyl alcohol, and ethyl acetate.

What is meant by the term "fine dispersion" is a system where one liquid is dispersed into a second liquid (bulk phase) that may or may not contain emulsifying agents and the dispersed droplets have an average droplet size less than 1 micron. Such fine dispersion systems may or may not be thermally stable. During the formation of the fine dispersion, solid particles may be formed. These solid particles may be dispersed in one or more phases in the system.

What is meant by the term "surface active compounds" are compounds such as an anionic surfactant, a cationic surfactant, a zwitterionic surfactant, a nonionic surfactant or a biological surface active molecule. The surface active compound can be added to the organic phase, the aqueous phase or to both the organic phase and the aqueous phase. The surface active compound should be present in an amount by weight of the aqueous phase or the organic phase, whatever the case may be, from less than about 1% to about 30%, more preferably from about 1 % to about 20%, or any range or combination of ranges therein.

Suitable anionic surfactants include but are not limited to: potassium laurate, sodium lauryl sulfate, sodium dodecylsulfate, alkyl polyoxyethylene sulfates, sodium alginate, dioctyl sodium sulfosuccinate, phosphatidyl choline, phosphatidyl glycerol, phosphatidyl inosine, phosphatidylserine, phosphatidic acid and their salts, glyceryl esters, sodium carboxymethylcellulose, cholic acid and other bile acids (e.g., cholic acid, deoxycholic acid, glycocholic acid, taurocholic acid, glycodeoxycholic acid) and salts thereof (e.g., sodium deoxycholate, etc.).

Suitable cationic surfactants include, but are not limited to, quaternary ammonium compounds, such .as benzalkonium chloride, cetyltrimethylammonium bromide, lauryldimethylbenzylammonium chloride, acyl carnitine hydrochlorides, or alkyl pyridinium halides. As anionic surfactants, phospholipids may be used. Suitable phospholipids include, for example phosphatidylcholine, phosphatidylethanolamine, phosphatidylserine, phosphatidyl inositol, phosphatidylglycerol, phosphatidic acid, lysophospholipids, egg or soybean phospholipid or a combination thereof. The phospholipid may be salted or desalted, hydrogenated or partially hydrogenated or natural, semisynthetic or synthetic.

Suitable nonionic surfactants include: polyoxyethylene fatty alcohol ethers (Macrogol and Brij), polyoxyethylene sorbitan fatty acid esters (Polysorbates), polyoxyethylene fatty acid esters (Myrj), sorbitan esters (Span), glycerol monostearate, polyethylene glycols, polypropylene glycols, cetyl alcohol, cetostearyl alcohol, stearyl alcohol, aryl alkyl polyether alcohols, polyoxyethylene-polyoxypropylene copolymers (poloxomers), polaxamines, methylcellulose, hydroxycellulose, hydroxy propylcellulose, hydroxy propylmethylcellulose, noncrystalline cellulose, polysaccharides including starch and starch derivatives such as hydroxyethylstarch (HES), polyvinyl alcohol, and polyvinylpyrrolidone. In a preferred form of the invention, the nonionic surfactant is a polyoxyethylene and polyoxypropylene copolymer and preferably a block copolymer of propylene glycol and ethylene glycol. Such polymers are sold under the tradename POLOXAMER also sometimes referred to as PLURONIC®, and sold by several suppliers including Spectrum Chemical and Ruger. Among polyoxyethylene fatty acid esters is included those having short alkyl chains. One example of such a surfactant is SOLUTOL® HS 15, polyethylene-660-hydroxystearate, manufactured by BASF Aktiengesellschaft.

Surface active biological molecules include such molecules as albumin, casein, heparin, hirudin, hetastarch or other appropriate biocompatible agents.

In a preferred form of the invention, the aqueous phase includes a protein as the surface active compound. A preferred protein is albumin. The protein may also function as an excipient. In embodiments in which protein is not the surface active compound, other excipients may be included in the multiphase system. Suitable excipients include, but are not limited to, saccharides, disaccharides, and sugar alcohols. A preferred disaccharide is sucrose, and a preferred sugar alcohol is mannitol.

### Removing a portion of the organic solvent in the multiphase system by sonication to form submicron sized particles

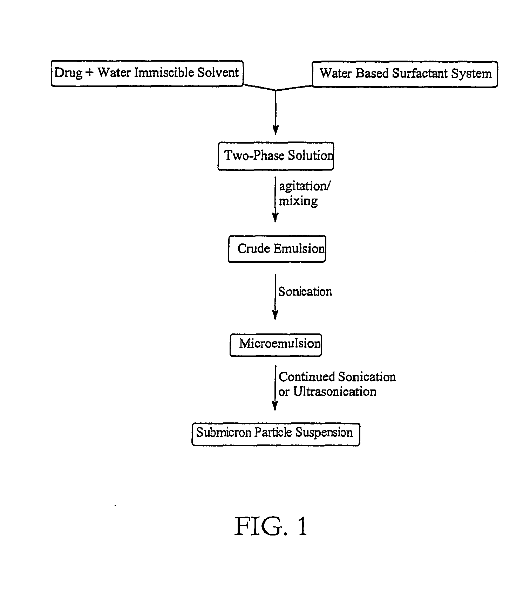

One method to form submicron sized particles in the present invention is to sonicate the multiphase system to evaporate a portion of the organic phase to cause precipitation of the compound as a suspension of particles in the aqueous phase (FIG. 1). The step of sonicating can be carried out with any suitable sonication device (Branson Model S-450A or Cole-Parmer 500/750 Watt Model). Such devices are well known in the industry. Typically the sonication device has a sonication horn or probe that is inserted into the multiphase system of interest to emit sonic energy into the solution. The sonicating device, in a preferred form of the invention, is operated at a frequency of from about 1 kHz to about 90 kHz and more preferably from about 20 kHz to about 40 kHz or any range or combination of ranges therein. The probe sizes can vary and preferably is in distinct sizes such as ½ inch or ¼ inch or the like. It may also be desirable to cool the solution during sonication to temperatures below room temperature. It may also be desirable to employ other mixing devices such as homogenizers, blenders or other stirring devices to assist in the process.

Exposing the dispersion droplets to shear energy can reduce the droplet sizes. Sonication provides a source of shear energy that effectively reduces the diameters of the dispersion droplets. Shear from sonication results from the compression and rarefaction of the propagation medium of the sound waves. In pure liquids this oscillation between compression and rarefaction is sufficiently energetic to cause cavitation, which is the tearing of the liquid to cause bubble formation. In a dispersion, the analogous process results in tearing the dispersed liquid particles into smaller particles. Cavitation and the warming of the dispersion during sonication also appear to effect removal of the water immiscible solvent. As the solvent is removed the solubility of the water-insoluble compound in the dispersion decreases, eventually allowing precipitation of the compound. Under appropriate conditions, the precipitation of the insoluble compound occurs in a manner which retains the original particle size of the sonicated dispersion.

The sonicating step is effective to remove nearly all solvent in the system to provide a particle suspension essentially free of the organic phase.

The present invention further contemplates additional processing of the resulting dispersion including removal of any residual solvent that may exist by means such as evaporation by the addition of heat or under reduced pressure, or through diafiltration. The solvent-free suspension can then be filtered through an appropriate 0.2 µm filter, resulting in a sterile suspension. This suspension is then amenable to further processing including freezing or lyophilization.

The particles of the pharmaceutically effective compound should be less than about 2 µm in diameter as determined by light scattering (HORIBA) or microscopic measurements. More preferably the particles should be less than about 1 µm, even more preferably less than about 400 nm and even more preferably less than about 200 nm and most preferably less than about 100 nm or any range or combination of ranges therein.

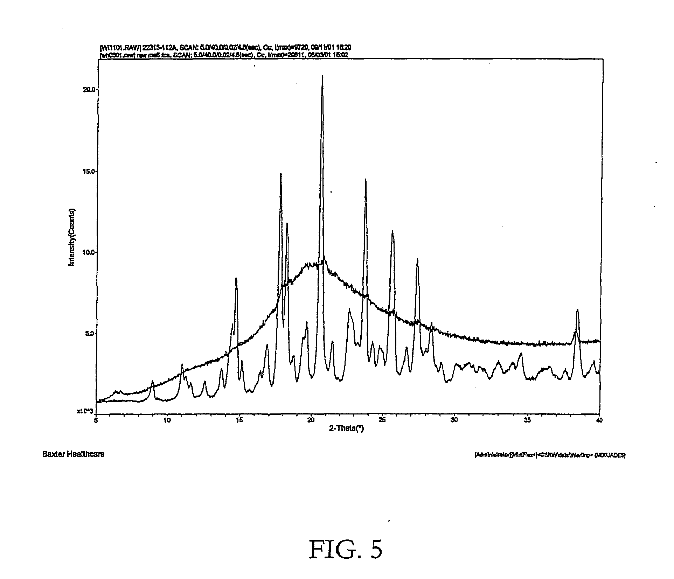

The particles have a generally spherical shape. Further, in a preferred form of the invention the particles will be amorphous. What is meant by amorphous is an X-ray crystal study of the particles shows virtual absence of x-ray peaks. See example 8 and FIG. 5.

### Formation of the fine dispersion

Another method to form submicron sized particles in the present invention is to remove the liquid phase of the multiphase system by freezing and lyophilizing a fine dispersion of the multiphase system. Fine dispersions can be formed from crude dispersions by energy addition. While various energy addition methods can be used to form the fine dispersion from the crude dispersion, the preferred methods are sonication and homogenization. In the methods using sonication, any suitable sonication device can be used. Examples of suitable sonication device include Branson Model S-450A or Cole-Parmer 500/750 Watt Model. Such devices are well known in the industry. Typically the sonication device has a sonication horn or probe that is inserted into the multiphase system of interest to emit sonic energy into the solution. The sonicating device, in a preferred form of the invention, is operated at a frequency of from about 1 kHz to about 90 kHz and more preferably from about 20 kHz to about 40 kHz or any range or combination of ranges therein. The probe sizes can vary and preferably is in distinct sizes such as 1 inch , ½ inch or ¼ inch or the like. It may also be desirable to cool the solution during sonication to temperatures below room temperature. In the methods using homogenization, any suitable homogenization device can be used. One example of such a device is the Avestin Emulsiflex-C5 homogenizer operating at about 5,000 psi to about 30,000 psi, and preferably from about 10,000 to 20,000 psi. In Example 11, an Avestin Emulsiflex-C5 homogenizer is used to form the fine dispersion. In this example, the crude dispersion is homogenized at 10,000 to 15,000 psi for 5 minutes in the temperature range of 23°C to 30°C. Other suitable energy addition methods to form the fine dispersion include, but are not limited to, high speed mixing, mechanical agitation, extrusion, microfluidization and other appropriate high shear techniques sufficient to provide dispersed droplets less than 2 microns.

Exposing the crude dispersion droplets to shear energy can reduce the droplet sizes to form a fine dispersion. Addition of energy to the crude dispersion by methods such as sonication or homogenization provides a source of shear energy that effectively reduces the diameters of the dispersion droplets. Shear forces from sonication or homogenization results from the compression and rarefaction of the propagation medium of the sound waves as well as mechanical shear from components of such systems. In pure liquids this oscillation between compression and rarefaction is sufficiently energetic to cause cavitation. In a dispersion, the analogous process results in tearing the dispersed liquid particles into smaller particles.

### Removing of the liquid phase of the fine dispersion

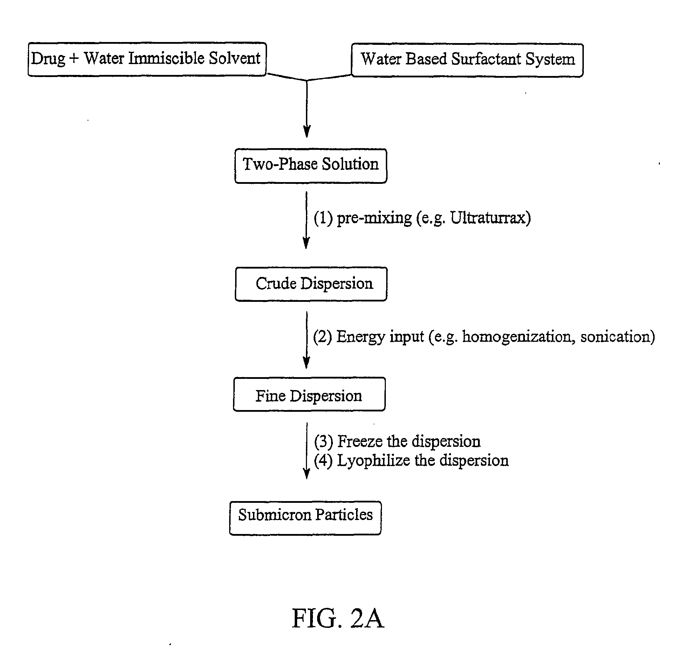

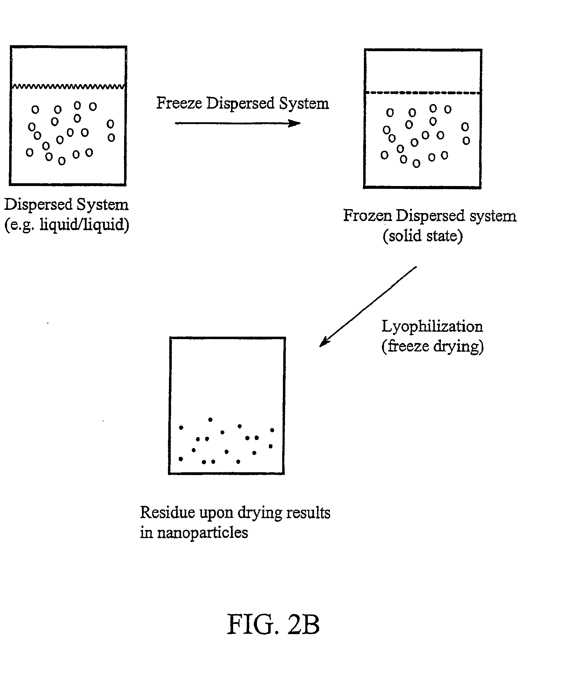

The present invention further removes the bulk liquid phase of the fine dispersion, including the remaining water immiscible organic solvent, to obtain the submicron sized particles. The sonicated or homogenized dispersion is first frozen to form a solid bulk state, which is then followed by lyophilization (e.g., using a Vertis Sentry bench model lyophilizer). (See FIG. 2). The solid product obtained upon lyophilization results in nanoparticles having a mean particle size of less than 500 nm in diameter as determined by light scattering (HORIBA) or microscopic measurements, and preferably less than 200 nm. The dry nanoparticles prepared in the present invention are preferably substantially free of any residual organic solvent from the organic phase of the multiphase system. The term "substantially free" means that the residual organic solvent present in the dry particles is substantially less than the level of solvent regarded as acceptable from a toxicological standpoint, for example, 5 ppm or less.

The particles generally have a spherical shape. Furthermore, in a preferred form of the invention the particles will be amorphous. What is meant by amorphous is an X-ray crystal study of the particles shows virtual absence of x-ray peaks.

### Sterilization of the fine dispersion and redispersing of the nanoparticles

In another preferred form of the invention, the fine dispersion is sterilized before being frozen. The preferred method of sterilization is sterile filtration using a 022 µm membrane filter. The nanoparticles obtained from lyophilization may further be redispersed in an appropriate aqueous dispersing medium, such as water, normal saline, buffered solutions, buffered saline, and the like. The redispersed nanoparticle suspension is now suitable for *in vivo* delivery by parenteral administration. Modes of parenteral administration include intravenous, intra-arterial, intrathecal, intraperitoneal, intraocular, intra-articular, intramuscular, subcutaneous injection, and the like. The preferred mode of parenteral administration is intravenous.

### Example 1: Preparation of a 0.5% itraconazole suspension using a 1:10 ratio of O/W

A 5% lecithin/glycocholate surfactant solution was prepared (100mL) and combined with 10 mL of a chloroform solution containing itraconazole (0.5 grams). The resulting mixture was manually shaken to generate a crude emulsion and set in an ice bath to chill. After cooling for 5 minutes the emulsion was sonicated every other minute for 10 minutes (5 minutes total sonication time at 40% power using a ½" probe at 20kHz) and then rotovapped at ~120 Torr (no heat) to remove the chloroform. The resulting solid particle dispersion was analyzed by light scattering detection (HORIBA) which revealed particles having a mean diameter of 97.78 nm.

### Example 2: Preparation of a 1.0% itraconazole suspension using a 1:5 ratio of O/W

A 5% lecithin/glycocholate surfactant solution was prepared (50 mL) and combined with 5 mL of a chloroform solution containing itraconazole (0.5 grams). The resulting mixture was manually shaken to generate a crude emulsion and set in an ice bath to chill. After cooling for 5 minutes the emulsion was sonicated every other minute for 10 minutes (5 minutes total sonication time) and then rotovapped at ~100 Torr (no heat) to remove the chloroform. The resulting solid particle dispersion was analyzed by light scattering detection (HORIBA) which revealed particles having a mean diameter of 135 nm.

### Example 3:

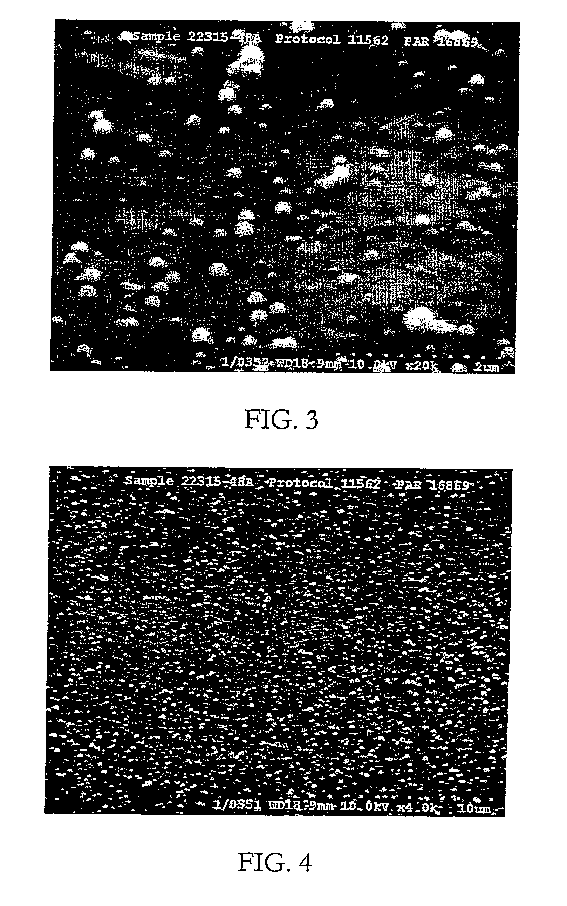

The process described in example 1 was repeated with the resulting particles having a mean diameter of 139 nm. This suspension was further analyzed by scanning electron microscopy to reveal solid spherical particles less than 200 nm in size. FIG. 3 reveals the spherical nature of the particles produced. The sample was prepared by filtration of a small portion o the suspension through a 80 nm filter and using standard SEM sample preparation techniques. Analysis of particles produced by this process revealed the particles to be completely amorphous as determined by x-ray powder diffraction (FIG. 4).

### Example 4: Preparation of a 1.0% itraconazole suspension using a 2:5 ratio of O/W

A 5% lecithin/sodium glycocholate solution was prepared (50 mL) and combined with 20 mL of chloroform containing itraconazole (0.5 grams). The resulting mixture was manually shaken to generate a crude emulsion and set in ice bath to chill. After cooling for 5 minutes the emulsion was sonicated every other minute for 8 minutes and than sonicated for 30 seconds (giving 4 minutes and 30 seconds of total sonication time) using a 1/2" probe at 40% amplitude. The sonicated dispersion was solvent evaporated at ~100 Torr (no heat) to remove chloroform. 10 mL of the final solution was filtered through a 0.2 micron filter. Both filtered and unfiltered solid particle dispersions were analyzed by light scattering detection (HORIBA), which revealed particles having a mean diameter of 110 nm and 106 nm respectively.

### Example 5:

A 5% lecithin/sodium glycocholate solution was prepared (50 mL) and combined with 10 mL of methylene chloride containing itraconazole (0.5 grams). The resulting mixture was manually shaken to generate a crude emulsion and set in ice bath to chill. After cooling for 5 minutes the emulsion was sonicated every other minute for 6 minutes (giving 3 minutes of total sonication time) using a 1/2" probe at 40% amplitude. The sonicated dispersion was solvent evaporated at ~100 Torr (no heat) to remove methylene chloride. The resulting solid particle dispersion was analyzed by light scattering detection (HORIBA) which revealed particles having a mean diameter of 144 nm.

### Example 6:

A 5% lecithin/sodium glycocholate solution was prepared (50 mL) and combined with 5 mL of methylene chloride containing itraconazole (0.5 grams). The resulting mixture was manually shaken to generate a crude emulsion and set in ice bath to chill. After cooling for 5 minutes the emulsion was sonicated every other 30 seconds for 6 minutes (giving 3 minutes of total sonication time) using a 1/4" probe at 20% amplitude. The sonicated solution was evaporated using rotavapor at ~100 Torr (no heat) to remove methylene chloride. Resulting solid particle dispersions was analyzed by light scattering detection (HORIBA) which revealed particles having a mean diameter of 109 nm.

### Example 7: Determination of solid particle size and morphology directly after sonication

The process described in example 6 was repeated except that no solvent removal was performed after sonication. Instead the sample was submitted for particle size determination by HORIBA analysis and scanning electron microscopy. HORIBA results indicated a mean particle diameter of 156 nm. The SEM pictures revealed solid spherical particles under 200 nm in size.

### Example 8: Determination of Amorphous Nature of Drug Particles

A crude itraconazole emulsion was prepared by combining 50 mL of a surfactant solution (2.2% lecithin, 0.5% sodium glycocholate, 1.0% polyvinylpyrrolidone) with 5 mL of a methylene chloride solution containing 0.5 grams of itraconazole. The mixture was then manually shaken to disperse the oil droplets into the surfactant matrix.

The crude emulsion was sonicated every other 30 seconds for 6 minutes using 1/4" probe at 20% amplitude and 20 kHz (temperature ~5°C using an ice bath). The sonicated solution was then rotovapped under house vacuum (100torr) for 15-20 minutes followed by 10 minutes under a high vacuum (<20 Torr). Part of the solution was stored at -70 degrees Celsius for about an hour, and subsequently lyophilized (>48 hours). Particle size of the remaining suspension was determined to be 168 nm by light scattering analysis (HORIBA). Inspection of the freeze-dried nanoparticles after lyophilization by visible light microscopy did not reveal any crystals present. Spherical particle halos were barely observable indicating that the itraconazole nanoparticles were intact.

The lyophilized itraconazole nanoparticles were assessed by X-ray powder diffraction and determined to be completely amorphous (virtual absence of x-ray peaks). In the raw material scan (the lower curve in FIG. 5) many peaks are observable revealing the crystalline nature of the compound in its original state.

### Example 9: Nanoparticles of itraconazole using sonication to create the fine dispersion

Itraconazole (0.5 grams) was dissolved in 3mL of methylene chloride and combined with 50 mL of a 5% albumin solution. The combined solutions were manually shaken to effect dispersion of the two liquids. The crude dispersion was than sonicated at 5°C for 6 minutes (sonicating every other 30 seconds using a 1/4" probe at 20% amplitude). The sonicated solution was frozen at -80°C and subsequently lyophilized. The lyophilized product was analyzed by light scattering detection (HORIBA), which revealed particles having a mean diameter of 187 nm.

### Example 10: Nanoparticles of cyclosporin using sonication to create the fine dispersion

The general process described in example 1 was repeated except cyclosporin was used in place of itraconzole. Final particle size was 185 nm (HORIBA light scattering analysis).

### Example 11: Dispersion lyophilization (using homogenization as the energy addition step)

Itraconazole (0.5 grams) was dissolved in 5.0 mL of dichloromethane and mixed with 50 mL of a 5% albumin solution. This was made into a crude dispersion by treatment with an Ultraturrax-T25 mixer for 10 seconds. The crude dispersion was homogenized at 10,000-12,000 psi using an Avestin Emulsiflex C5 homogenizer for 6 passes in the temperature range of 18°C to 27°C to form a fine dispersion. The fine dispersion was immediately frozen at -80°C and lyophilized without temperature control to produce a fluffy powder. The powder was reconstituted with water and the solid particle dispersion analyzed by light scattering detection (HORIBA) which revealed itraconazole particles having a mean diameter of 122 nm.

### Example 12: Sterile filtration incorporated into the dispersion/lyophilization process

Itraconazole (0.5 grams) was dissolved in 5 mL of dichloromethane and combined with 50 mL of a 5% albumin solution. The contents were then made into a crude dispersion by treatment with an Ultraturrax-T25 mixer for 10 seconds. Homogenization of the crude dispersion at 10,000 to 15,000 psi (Avestin Emulsiflex C5 homogenizer) for 5 minutes in the temperature range of 23 °C to 30°C resulted in a microdroplet or fine dispersion. This microdroplet dispersion was easily filtered through a 0.22 µm membrane filter. Inspection of the filter did not reveal the presence of any drug material. The filtered dispersion was then frozen at -80° C. After several hours the frozen dispersion system was lyophilized without temperature control to produce a fluffy powder. The resulting lyophilized cake, containing solid itraconazole particles, was reconstituted with water and the dispersion analyzed by light scattering detection (HORIBA) to reveal itraconazole particles with a mean diameter of 144 nm.

### Example 13: Incorporation of sodium deoxycholate into the process

50 mL of 5% albumin solution, 2 mL of a 2.75 % sodium deoxycholate solution and 3.5 mL of a solution of itraconazole in dichloromethane containing 0.5 grams of itraconazole were mixed in a 100 mL beaker and treated with an Ultraturrax-T25 mixer for 10 seconds at 11,000 rpm. The crude dispersion was homogenized at 15,000 to 20,000 psi at room temperature to form a fine dispersion. The fine dispersion was transferred to a 250 mL glass bottle and frozen immediately at -80°C. The frozen sample was lyophilized to produce submicron particles. The lyophilized product was reconstituted with water and analyzed by light scattering detection (HORIBA), which revealed particles having a mean diameter of 207 nm.

From the foregoing, it will be observed that numerous variations and modifications may be effected without departing from the spirit and scope of the invention. It is to be understood that no limitation with respect to the specific apparatus illustrated herein is intended or should be inferred. It is, of course, intended to cover by the appended claims all such modifications as fall within the scope of the claims.

Various aspects of the invention are described in the following numbered paragraphs.
1. A process for preparing submicron sized particles comprising the steps of:
   providing a multiphase system having an organic phase and an aqueous phase, the organic phase having a pharmaceutically active compound therein; and
   sonicating the system to evaporate a portion of the organic phase to cause precipitation of the compound in the aqueous phase and having an average effective particle size of less than about 2µm.
2. The process of paragraph 1, wherein the ratio by weights of the organic phase to the aqueous phase is from about 1:99 to about 99:1.
3. The process of paragraph 1, wherein the compound is present in an amount by weight of the organic phase from less than about 1 % to about 40%.
4. The process of paragraph 1, wherein the compound is present in an amount by weight of the organic phase from less than about 1 % to about 25%.
5. The process of paragraph 1, wherein the compound is present in an amount by weight of the organic phase from less than about 1% to about 10%.
6. The process of paragraph 1, wherein the compound has a water solubility of less than 10 mg/ml.
7. The process of paragraph 1, wherein the compound has a water solubility of less than 8 mg/ml.
8. The process of paragraph 1, wherein the step of sonicating the system comprises the steps of:
   providing a sonication device having a transducer for emitting sonic energy; and
   exposing the system to said sonic energy sufficient to allow for cavitation to occur.
9. The process of paragraph 8, wherein the step of sonicating comprises the steps of: operating the device at a frequency of from about 1 kHz to about 90 kHz.
10. The process of paragraph 1, further comprising the step of adding a surface active compound to either the organic phase, the aqueous phase or to both the organic phase and the aqueous phase.
11. The process of paragraph 10, wherein the surface active compound is selected from the group consisting of anionic surfactants, cationic surfactants, zwitterionic surfactants, nonionic surfactants and biological surface active molecules.
12. The process of paragraph 11, wherein the nonionic surfactant is selected from the group consisting of: polyoxyethylene fatty alcohol ethers, polyoxyethylene sorbitan fatty acid esters, polyoxyethylene fatty acid esters, sorbitan esters, glycerol monostearate, polyethylene glycols, polypropylene glycols, cetyl alcohol, cetostearyl alcohol, stearyl alcohol, aryl alkyl polyether alcohols, polyoxyethylene-polyoxypropylene copolymers, polaxamines, methylcellulose, hydroxycellulose, hydroxy propylcellulose, hydroxy propylmethylcellulose, noncrystalline cellulose, polysaccharides, starch, starch derivatives, hydroxyethyistarch, polyvinyl alcohol, and polyvinylpyrrolidone.
13. The process of paragraph 11, wherein the anionic surfactant is selected from the group consisting of : anionic surfactant is selected from the group consisting of : potassium laurate, triethanolamine stearate, sodium lauryl sulfate, sodium dodecylsulfate, alkyl polyoxyethylene sulfates, sodium alginate, dioctyl sodium sulfosuccinate, phosphatidyl glycerol, phosphatidyl inositol, phosphatidylserine, phosphatidic acid and their salts, glyceryl esters, sodium carboxymethylcellulose, bile acids and their salts, cholic acid, deoxycholic acid, glycocholic acid, taurocholic acid, glycodeoxycholic acid, and calcium carboxymethylcellulose.
14. The process of paragraph 11, wherein the cationic surfactant is selected from the group consisting of quaternary ammonium compounds, benzalkonium chloride,cetyltrimethyl ammonium bromide, chitosans and lauryldimethylbenzylammonium chloride.
15. The process of paragraph 11, wherein the surface active biological molecules are selected from the group consisting of: albumin, casein, heparin, and hirudin.
16. The process of paragraph 1, further comprising the step of: adding a phospholipid to either the organic phase, the aqueous phase or to both the organic phase and the aqueous phase.
17. The process of paragraph 16, wherein the phospholipid is selected from natural phospholipids and/or synthetic phospholipids.
18. The process of paragraph 16, wherein the phospholipid is selected from the group consisting of: phosphatidylcholine, phosphatidylethanolamine, phosphatidylserine, phosphatidylinositol, phosphatidylglycerol, phosphatidic acid, lysophospholipids, egg phospholipid and soybean phospholipid.
19. The process of paragraph 16, wherein further comprising the step of adding a surface-active compound to the system.
20. The process of paragraph 19, wherein the surface active compound is selected from the group consisting of anionic surfactants, cationic surfactants, zwitterionic surfactants, nonionic surfactants, and biological surface-active molecules.
21. The process of paragraph 20, wherein the nonionic surfactant is selected from the group consisting of: polyoxyethylene fatty alcohol ethers, sorbitan fatty acid esters, polyoxyethylene fatty acid esters, sorbitan esters, glycerol monostearate, polyethylene glycols, cetyl alcohol, cetostearyl alcohol, stearyl alcohol,poloxamers, polaxamines, methylcellulose, hydroxycellulose, hydroxy propylcellulose, hydroxy propylmethylcellulose, noncrystalline cellulose, polyvinyl alcohol, polyvinylpyrrolidone, albumin, heparin, and hirudin.
22. The process of paragraph 20, wherein the anionic surfactant is selected from the group consisting of: potassium laurate, triethanolamine stearate, sodium lauryl sulfate, sodium dodecylsulfate, alkyl polyoxyethylene sulfates, sodium alginate, dioctyl sodium sulfosuccinate, phosphatidyl glycerol, phosphatidyl inositol, phosphatidylserine, phosphatidic acid and their salts, glyceryl esters, sodium carboxymethylcellulose, bile acids and their salts, cholic acid, deoxycholic acid, glycocholic acid, taurocholic acid, glycodeoxycholic acid, and calcium carboxymethylcellulose.
23. The process of paragraph 20, wherein the cationic surfactant is selected from the group consisting of quaternary ammonium compounds, benzalkonium chloride, cetyltrimethylammonium bromide, chitosans and lauryldimethylbenzylammonium chloride.
24. The process of paragraph 20, wherein the biological surface active molecules are selected from the group consisting of: albumin, casein, heparin, and other proteins.
25. The process of paragraph 1, wherein the organic phase comprises a water immiscible solvent.
26. The process of paragraph 25, wherein the water immiscible solvent is selected from the group consisting of: is selected from the group consisting of : linear, branched or cyclic alkanes with carbon number of 5 or higher, linear, branched or cyclic alkenes with carbon number of 5 or higher, linear, branched or cyclic alkynes with carbon number of 5 or higher; aromatic hydrocarbons completely or partially halogenated hydrocarbons, ethers, esters, ketones, mono-, di-or tri-glycerides, native oils, alcohols, aldehydes, acids, amines, linear or cyclic silicones, hexamethyldisiloxane, or any combination of these solvents.
27. The process of paragraph 26, wherein the water immiscible solvent has a vapor pressure higher than water at room temperature.
28. The process of paragraph 1, wherein generation of the multiphase system is accomplished by use of piston gap homogenizers, colloidal mills, high speed stirring, extrusion, manual agitation or shaking, microfluidization, or other high shear conditions.
29. The process of paragraph 1, wherein the compound is selected from the group consisting of: antihyperlipidemics, anesthetics,antiasthamatics, antimicrobials, antifungals, antineoplastics, non-steroidal anti-inflammatory drugs, antihypercholesteremic agents, analgesics, steroidal compounds,antipyretics, antidepressants, antiarrhthmics, antianxiety drugs, antimanics, antiarthritics, antihistamines, anti-infectives, water insoluble vitamins, antipsychotics, sedatives, antihypertensive agents, diagnostic agents, anticonvulsants and immunosuppresants.
30. A process for preparing an aqueous suspension of submicron sized particles comprising the stepsof : providing an organic phase of a pharmacologically active compound dissolved in a water immiscible solvent; providing an aqueous phase; combining the organic phase with the aqueous phase to form a crude dispersion; and sonicating the crude dispersion to cause precipitation of the compound as a suspension of particles in the aqueous phase wherein the aqueous phase is essentially free of the water immiscible solvent.
31. The process of paragraph 30, wherein the particle is in an amorphous form.
32. The process of paragraph 30, wherein the particle has an average effective particle size of less than about 2um.
33. The process of paragraph 30, wherein the particle has an average effective particle size of less than about 400nm.
34. The process of paragraph 30, wherein the particle has an average effective particle size of less than about 300nm.
35. A process for preparing submicron sized particles comprising the steps of:
   providing a crude dispersion of a multiphase system having an organic phase and an aqueous phase, the organic phase having a pharmaceutically active compound therein;
   providing energy to the crude dispersion to form a fine dispersion;
   freezing the fine dispersion; and
   lyophilizing the frozen dispersion to obtain particles having a mean particle size of less than 500nm.
36. The process of paragraph 35, wherein the particles have a mean particle size of less than 200 nm.
37. The process of paragraph 35, wherein the particles are substantially free of the organic phase.
38. The process of paragraph 35, wherein the crude dispersion or the fine dispersion includes solid particles in one or more phases in the multiphase system.
39. The process of paragraph 35, wherein the compound has a water solubility of less than 10mg/ml.
40. The process of paragraph 35, wherein the compound has a water solubility of less than 8 mg/ml.
41. The process of paragraph 35, wherein the multiphase system is an oil in water (O/W) emulsion.
42. The process of paragraph 35, wherein the multiphase system is a water in oil (W/O) emulsion.
43. The process of paragraph 35, wherein the multiphase system is a water in oil in water(W/O/W) emulsion.
44. The process of paragraph 35, wherein the multiphase system is an oil in water in oil (O/W/O) emulsion.
45. The process of paragraph 35, wherein the ratio by weights of the organic phase to the aqueous phase is from about 1:99 to about 99:1.
46. The process of paragraph 35, wherein the ratio by weights of the organic phase to the aqueous phase is from about 1:99 to about 40:60.
47. The process of paragraph 35, wherein the ratio by weights of the organic phase to the aqueous phase is from about 2:98 to about 30:70.
48. The process of paragraph 35, wherein the compound is present in an amount by weight of the organic phase from less than about 1 % to about 40%.
49. The process of paragraph 35, wherein the compound is present in an amount by weight of the organic phase from less than about 1 % to about 25%.
50. The process of paragraph 35, wherein the compound is present in an amount by weight of the organic phase from less than about 1% to about 10%.
51. The process of paragraph 35, wherein the method of providing the crude dispersion is selected from the group consisting of: shaking, agitating, vortexing, mixing, and stirring.
52. The process of paragraph 35, wherein the step of providing energy to the crude dispersion comprises the steps of:
   providing a sonication device having a transducer for emitting sonic energy; and
   exposing the system to said sonic energy sufficient to allow for cavitation to occur.
53. The process of paragraph 52, wherein the sonication device is operating at a frequency of from about 1 kHz to about 90 kHz.
54. The process of paragraph 52, wherein the sonication device is operating at a frequency of from about 20 kHz to about 40 kHz.
56. The process of paragraph 35, wherein the step of providing energy to the crude dispersion is by homogenization.
57. The process of paragraph 56, wherein the crude dispersion is homogenized at about 5,000 to 30,000 psi.
58. The process of paragraph 56, wherein the crude dispersion is homogenized at about 10,000 to 15,000 psi.
59. The process of paragraph 56, wherein the crude dispersion is homogenized at about 15,000 to 20,000 psi.
60. The process of paragraph 35, further comprises the step of adding a surface active compound to either the organic phase, the aqueous phase or to both the organic phase and the aqueous phase.
61. The process of paragraph 60, wherein the surface active compound is selected from the group consisting of nonionic surfactants, anionic surfactants, cationic surfactants, zwitterionic surfactants and biological surface active molecules.
62. The process of paragraph 61, wherein the nonionic surfactant is selected from the group consisting of: polyoxyethylene fatty alcohol ethers, sorbitan fatty acid esters, polyoxyethylene fatty acid esters, sorbitan esters, glycerol monostearate, polyethylene glycols, cetyl alcohol, cetostearyl alcohol, stearyl alcohol, poloxamers, polaxamines, methylcellulose, hydroxycellulose, hydroxy propylcellulose, hydroxy propylmethylcellulose, noncrystalline cellulose, polyvinyl alcohol, polyvinylpyrrolidone, albumin, heparin, and hirudin.
63. The process of paragraph 61, wherein the anionic surfactant is selected from the group consisting of: potassium laurate, triethanolamine stearate, sodium lauryl sulfate, sodium dodecylsulfate, alkyl polyoxyethylene sulfates, sodium alginate, dioctyl sodium sulfosuccinate, phosphatidyl glycerol, phosphatidyl inositol, phosphatidylserine, phosphatidic acid and their salts, glyceryl esters, sodium carboxymethylcellulose, bile acids and their salts, cholic acid, deoxycholic acid, glycocholic acid, taurocholic acid, glycodeoxycholic acid, and calcium carboxymethylcellulose.
64. The process of paragraph 61, wherein the cationic surfactant is selected from the group consisting of quaternary ammonium compounds, benzalkonium chloride, cetyltrimethylammonium bromide, chitosans and lauryldimethylbenzylammonium chloride.
65. The process of paragraph 61, wherein the biological surface active molecule is selected from the group consisting of : albumin, casein, heparin, and hirudin.
66. The process of paragraph 61, wherein the surface active compound is albumin.
67. The process of paragraph 35, further comprising the step of adding an excipient to the multiphase system.
68. The process of paragraph 67, wherein the excipient is selected from the group consisting of proteins, saccharides, diaccharides, and sugar alcohols.
69. The process of paragraph 68, wherein the disaccharide is sucrose.
70. The process of paragraph 68, wherein the sugar alcohol is mannitol.
71. The process of paragraph 61, wherein the surface active compound is present in an amount by weight of the aqueous phase or the organic phase from less than about 1 % to about 30%.
72. The process of paragraph 61, wherein the surface active compound is present in an amount by weight of the aqueous phase or the organic phase from less than about 1 % to about 20%.
73. The process of paragraph 35, wherein the organic phase comprises a water immiscible solvent.
74. The process of paragraph 73, wherein the water immiscible solvent is selected from the group consisting of: linear, branched or cyclic alkanes with carbon number of 5 or higher, linear, branched or cyclic alkenes with carbon number of 5 or higher, linear, branched or cyclic alkynes with carbon number of 5 or higher; aromatic hydrocarbons completely or partially halogenated hydrocarbons, ethers, esters, ketones, mono-, di-or tri-glycerides, native oils, alcohols, aldehydes, acids, amines, linear or cyclic silicones, hexamethyldisiloxane, or any combination of these solvents.
75. The process of paragraph 73, wherein the water immiscible solvent has a vapor pressure higher than water at room temperature.
76. The process of paragraph 35, wherein the organic phase comprises a partially water miscible solvent.
77. The process of paragraph 76, wherein the partially water miscible solvent is selected from the group consisting of : fluorinated solvents, tetrahydrofuran, propylene carbonate, benzyl alcohol, and ethyl acetate.
78. The process of paragraph 35, wherein the organic phase further includes a co-solvent.
79. The process of paragraph 78, wherein the co-solvent is a water miscible organic solvent.
80. The process of paragraph 35, wherein the compound is selected from the group consisting of: antihyperlipidemics, anesthetics, antiasthamatics, antimicrobials, antifungals, antineoplastics, non-steroidal anti-inflammatory drugs, antihypercholesteremic agents, analgesics, steroidal compounds, antipyretics, antidepressants, antiarrhthmics, antianxiety drugs, antimanics, antiarthritics, antihistamines, anti-infectives, water insoluble vitamins, antipsychotics, sedatives, antihypertensive agents, diagnostic agents, anticonvulsants and immunosuppresants.
81. The process of paragraph 35, further comprising the step of sterile filtering the fine dispersion prior to freezing and lyophilizing to produce the particles.
82. The process of paragraph 35, further comprising the step of redispersing the lyophilized particles in an aqueous medium.
83. The process of paragraph 82, wherein the redispersed nanoparticles are suitable for in vivo delivery by parenteral administration.
84. A process for preparing submicron sized particles comprising the steps of:
   providing an organic phase of a pharmacologically active compound dissolved in a water immiscible solvent;
   providing an aqueous phase with a surface active compound;
   combining the organic phase with the aqueous phase to form a crude dispersion; and
   providing energy to the crude dispersion to form a fine dispersion;
   freezing the fine dispersion;
   lyophilizing the frozen dispersion to obtain particles having a mean particle size of less than 500nm.
85. The process of paragraph 84, wherein the particles have a mean particle size of less than 200 nm.
86. The process of paragraph 84, wherein the surface active compound is albumin.
87. The process of paragraph 84, wherein the particles are substantially free of the organic phase.
88. The process of paragraph 84, wherein the method of forming the crude dispersion is selected from the group consisting of shaking, agitating, vortexing, mixing, and stirring.
89. The process of paragraph 84, wherein the method of providing energy to the crude dispersion to form the dispersion is selected from the group consisting of sonication and homogenization.
90. The process of paragraph 84, further comprising the step of sterile filtering the fine dispersion prior to freezing and lyophilizing to obtain the particles.
91. The process of paragraph 84, further comprising the step of redispersing the particles in an aqueous medium.
92. The process of paragraph 91, wherein the redispersed particles are suitable forii7 vivo delivery by parenteral administration.
93. A process for preparing an aqueous suspension of submicron sized particles comprising the steps of:
   providing an organic phase of a pharmacologically active compound dissolved in a water immiscible solvent;
   providing an aqueous phase with a surface active compound;
   combining the organic phase with the aqueous phase to form a crude dispersion;
   providing energy to the crude dispersion to form a fine dispersion;
   sterile filtering the fine dispersion;
   freezing the sterile filtered dispersion;
   lyophilizing the frozen dispersion to obtain particles having a mean particle size of less than 500nm; and
   redispersing the particles in an aqueous medium.
94. The process of paragraph 93, wherein the particles have a mean particle size of less than 200 nm.
95. The process of paragraph 93, wherein the particles are substantially free of the organic phase.
96. The process of paragraph 93, wherein the surface active compound is albumin.

## Claims

1. A process for preparing submicron sized particles comprising the steps of:
providing a crude dispersion of a multiphase system having an organic phase and an aqueous phase, the organic phase having a pharmaceutically active compound therein;
providing energy to the crude dispersion to form a fine dispersion;
freezing the fine dispersion; and
lyophilizing the frozen dispersion to obtain particles having a mean particle size of less than 500nm.

2. The process of claim 1, wherein the particles have a mean particle size of less than 200 nm.

3. The process of claim 1, wherein the particles are substantially free of the organic phase.

4. The process of claim 1, wherein the crude dispersion or the fine dispersion includes solid particles in one or more phases in the multiphase system.

5. The process of claim 1, wherein the compound has a water solubility of less than 10mg/ml, preferably less than 8 mg/ml.

6. The process of claim 1, wherein the multiphase system is an oil in water (O/W) emulsion, or a water in oil (W/O) emulsion, or a water in oil in water (W/O/W) emulsion, or an oil in water in oil (O/W/O) emulsion.

7. The process of claim 1, wherein the ratio by weights of the organic phase to the aqueous phase is from about 1:99 to about 99:1, preferably from about 1:99 to about 40:60, more preferably from about 2:98 to about 30:70.

8. The process of claim 1, wherein the compound is present in an amount by weight of the organic phase from less than about 1% to about 40%, preferably from less than about 1 % to about 25%, more preferably from less than about 1% to about 10%.

9. The process of claim 1, wherein the method of providing the crude dispersion is selected from the group consisting of: shaking, agitating, vortexing, mixing, and stirring.

10. The process of claim 1, wherein the step of providing energy to the crude dispersion comprises the steps of:
providing a sonication device having a transducer for emitting sonic energy; and
exposing the system to said sonic energy sufficient to allow for cavitation to occur.

11. The process of claim 10, wherein the sonication device is operating at a frequency of from about 1 kHz to about 90 kHz, preferably from about 20 kHz to about 40 kHz.

12. The process of claim 1, wherein the step of providing energy to the crude dispersion is by homogenization.

13. The process of claim 12, wherein the crude dispersion is homogenized at about 5,000 to 30,000 psi, preferably at about 10,000 to 15,000 psi, or at about 15,000 to 20,000 psi.

14. The process of claim 1, further comprises the step of adding a surface active compound to either the organic phase, the aqueous phase or to both the organic phase and the aqueous phase.

15. The process of claim 14, wherein the surface active compound is selected from the group consisting of nonionic surfactants, anionic surfactants, cationic surfactants, zwitterionic surfactants and biological surface active molecules.

16. The process of claim 15, wherein the nonionic surfactant is selected from the group consisting of : polyoxyethylene fatty alcohol ethers, sorbitan fatty acid esters, polyoxyethylene fatty acid esters, sorbitan esters, glycerol monostearate, polyethylene glycols, cetyl alcohol, cetostearyl alcohol, stearyl alcohol, poloxamers, polaxamines, methylcellulose, hydroxycellulose, hydroxy propylcellulose, hydroxy propylmethylcellulose, noncrystalline cellulose, polyvinyl alcohol, polyvinylpyrrolidone, albumin, heparin, and hirudin.

17. The process of claim 15, wherein the anionic surfactant is selected from the group consisting of : potassium laurate, triethanolamine stearate, sodium lauryl sulfate, sodium dodecylsulfate, alkyl polyoxyethylene sulfates, sodium alginate, dioctyl sodium sulfosuccinate, phosphatidyl glycerol, phosphatidyl inositol, phosphatidylserine, phosphatidic acid and their salts, glyceryl esters, sodium carboxymethylcellulose, bile acids and their salts, cholic acid, deoxycholic acid, glycocholic acid, taurocholic acid, glycodeoxycholic acid, and calcium carboxymethylcellulose.

18. The process of claim 15, wherein the cationic surfactant is selected from the group consisting of quaternary ammonium compounds, benzalkonium chloride, cetyltrimethylammonium bromide, chitosans and lauryldimethylbenzylammonium chloride.

19. The process of claim 15, wherein the biological surface active molecule is selected from the group consisting of: albumin, casein, heparin, and hirudin.

20. The process of claim 15, wherein the surface active compound is albumin.

21. The process of claim 1, further comprising the step of adding an excipient to the multiphase system.

22. The process of claim 21, wherein the excipient is selected from the group consisting of proteins, saccharides, disaccharides, and sugar alcohols.

23. The process of claim 22, wherein the disaccharide is sucrose.

24. The process of claim 22, wherein the sugar alcohol is mannitol.

25. The process of claim 15, wherein the surface active compound is present in an amount by weight of the aqueous phase or the organic phase from less than about 1 % to about 30%, preferably from less than about 1 % to about 20%.

26. The process of claim 1, wherein the organic phase comprises a water immiscible solvent.

27. The process of claim 26, wherein the water immiscible solvent is selected from the group consisting of: linear, branched or cyclic alkanes with carbon number of 5 or higher, linear, branched or cyclic alkenes with carbon number of 5 or higher, linear, branched or cyclic alkynes with carbon number of 5 or higher; aromatic hydrocarbons, completely or partially halogenated hydrocarbons, ethers, esters, ketones, mono-, di-or tri-glycerides, native oils, alcohols, aldehydes, acids, amines, linear or cyclic silicones, hexamethyldisiloxane, or any combination of these solvents.

28. The process of claim 26, wherein the water immiscible solvent has a vapor pressure higher than water at room temperature.

29. The process of claim 1, wherein the organic phase comprises a partially water miscible solvent.

30. The process of claim 29, wherein the partially water miscible solvent is selected from the group consisting of: fluorinated solvents, tetrahydrofuran, propylene carbonate, benzyl alcohol, and ethyl acetate.

31. The process of claim 1, wherein the organic phase further includes a co-solvent.

32. The process of claim 31, wherein the co-solvent is a water miscible organic solvent.

33. The process of claim 1, wherein the compound is selected from the group consisting of: antihyperlipidemics, anesthetics, antiasthmatics, antimicrobials, antifungals, antineoplastics, non-steroidal anti-inflammatory drugs, antihypercholesteremic agents, analgesics, steroidal compounds, antipyretics, antidepressants, antiarrhythmics, antianxiety drugs, antimanics, antiarthritics, antihistamines, anti-infectives, water insoluble vitamins, antipsychotics, sedatives, antihypertensive agents, diagnostic agents, anticonvulsants and immunosuppresants.

34. The process of claim 1, further comprising the step of sterile filtering the fine dispersion prior to freezing and lyophilizing to produce the particles.

35. The process of claim 1, further comprising the step of redispersing the lyophilized particles in an aqueous medium.

36. The process of claim 35, wherein the redispersed nanoparticles are suitable for in *vivo* delivery by parenteral administration.
